# EUROPEAN PATENT APPLICATION

(11) **EP 1 920 779 A1**
(43) Date of publication of application: **14.05.2008**
(21) Application number: 06782632.1
(22) Date of filing: 10.08.2006
(51) Int. Cl.: A61K 38/00, A61K 9/08, A61K 47/02, A61K 47/10, A61K 47/16, A61K 47/20, A61K 47/34, A61K 47/44

(54) **TECHNIQUE FOR STABILIZATION OF YW753 REPARATION**

(30) Priority: 12.08.2005 JP 2005234256
(71) Applicant: Astellas Pharma Inc., Tokyo 103-8411 (JP)
(72) Inventor: TODA, Atsushi, Chuo-ku Tokyo, 1038411 (JP); AOKI, Hiroshi, Chuo-ku Tokyo, 103-8411 (JP); KOJIMA, Seiki, Chuo-ku Tokyo, 1038411 (JP); NAKATA, Katsunori, Chuo-ku Tokyo, 103-8411 (JP)
(74) Representative: HOFFMANN EITLE
(86) International application number: PCT/JP2006/315838
(87) International publication number: WO 2007/020871

(57) **Abstract**

[Problems] An injection preparation containing a depsipeptide having a disulfide bond in its molecule, which can be easily prepared, and which is stable, is provided.

[Means for Solution] An injection preparation having a long-term stability can be provided by compounding a substance such as ferric chloride, making the solution to have a pH which is not higher than 7.0 and which is not lower than 1.0, and carrying out nitrogen replacement.

[Advantages of the Invention] The injection solution of the present invention is expected to provide an injection preparation in which formation of aggregate and cluster of a depsipeptide having a double bond in its molecule is avoided and which is excellent in a storage ability.

## Description

### TECHNICAL FIELD

The present invention relates to a depsipeptide-containing injection solution having a pH which is not higher than 7.0 and which is not lower than 1.0, comprising
(1) a depsipeptide compound having a disulfide bond in its molecule,
(2) a solubilizing agent,
(3) ferric chloride and/or copper sulfate and
(4) dithiothreitol in place of (3). Also, it relates to a depsipeptide-containing injection preparation, wherein the injection solution is filled in a container and the space is replaced by nitrogen; as well as to a method for preparing the same.

### BACKGROUND ART

It has been reported that, in protein, bonding of an intramolecular disulfide of fibroblast growth factor which is a protein derived from blood vessel (FGF-1) by oxidation of copper ion or iron ion takes place, resulting in formation of dimers of the protein (Non-Patent Document 1). It has been also reported that, in synthetic compounds, an SH group strongly participates in aggregation and polymerization and that the position, etc. of the SH group has some influences on the polymerization, etc. of the synthetic compound (Non-Patent Document 2).

Since dimers and polymers are considered to be foreign substances when administered to humans, it is necessary to avoid formation of them. Although production of dimers and polymers of proteins and synthetic compounds has been reported, it has been also known that the substances having a disulfide bond or an SH bond do not always result in aggregation and polymerization and that they are phenomena which are specific to each substance. Accordingly, it is the current status that no uniform means dealing with such aggregation and polymerization has been established yet.

A depsipeptide compound having a disulfide bond in its molecule increases the expression of cancer-suppressive gene by suppressing a histone deacetylating enzyme and, due to its versatile pharmacological actions such as inhibition of cell growth, induction of cell death, inhibition of angiogenesis, normalization of morphology and induction of differentiation, it may be said to be a semisynthetic compound of peptide having a novel functional mechanism whereby a strong antitumor action is expected (Patent Document 1 and Patent Document 2). Injection preparations containing the depsipeptide compound which have been known up to now are freeze-dried preparations produced by dissolving the depsipeptide compound in an organic solvent, etc. followed by subjecting to a freeze-drying. Accordingly, when troublesomeness during the manufacture of the freeze-dried preparation, troublesomeness in preparing upon actual use such as dissolving at the stage of administration, etc. are taken into consideration, there has been a demand for providing an injection solution as well as an injection preparation containing the depsipeptide compound being easily manufactured and also stable.

Patent Document 1: Pamphlet of the International Publication WO 00/42062
Patent Document 2: Gazette of Japanese Patent Laid-Open No. 2001/316283
Non-Patent Document 1: Kurt A. Engleka, et al. "Inactivation of Human Fibroblast Growth Factor-1 (FGF-1) Activity by Interaction with Copper Ions Involves FGF-1 Dimer Formation Induced by Copper-Catalyzed Oxidation", The Journal of Biological Chemistry, 1992, Vol. 267, No. 16, pages 11307 to 11315
Non-Patent Document 2: Toru Sasaki, et al. "Synthesis, Bioactivity and Cloning of the L-Type Calcium Channel Blocker ω-Conotoxin TxVII, Biochemistry, 1999, 38, pages 12876 to 12884)

### DISCLOSURE OF THE INVENTION

During the course of studies on injection preparations containing a compound represented by the following formula (I) (hereinafter, referred to as "Compound A") which is a depsipeptide compound having a disulfide bond in its molecule and is sparingly soluble in water, the present inventors have found a phenomenon that, when an injection preparation is produced by using the Compound A dissolved in propylene glycol or the like and is allowed to stand, sparingly soluble aggregate and polymer are produced. Another phenomenon where a quantitative value of the Compound A lowers when pH becomes high whereby the injection solution becomes unstable has been also found. When the investigation has been conducted for avoiding the production of the aggregate or polymer, it has been found that,only specific oxidizing agents and reducing agents are effective for avoidance of the production of aggregate and polymer and that, when the pH of the liquid is made not higher than 7.0 and not lower than 1.0 by an acidic substance such as hydrochloric acid, sulfuric acid and citric acid, stability upon storage for longer period of time can be ensured, resulting in accomplishment of the present invention.

Compound A 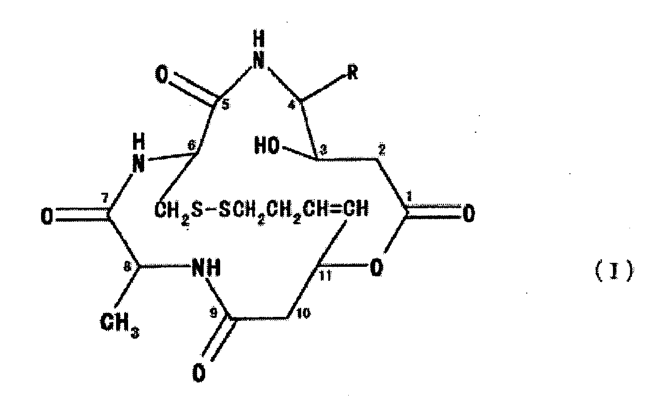 (In the formula, R is an isopropyl group, a sec-butyl group or an isobutyl group.)

Thus, the present invention relates to:
1. A depsipeptide-containing injection solution having a pH which is not higher than 7.0 and is not lower than 1.0, comprising
   (1) a depsipeptide compound having a disulfide bond in its molecule,
   (2) a solubilizing agent,
   (3) ferric chloride and/or copper sulfate, and
   (4) dithiothreitol in place of (3).
2. The depsipeptide-containing injection solution according to claim 1, wherein the depsipeptide compound is a compound represented by the formula (I): 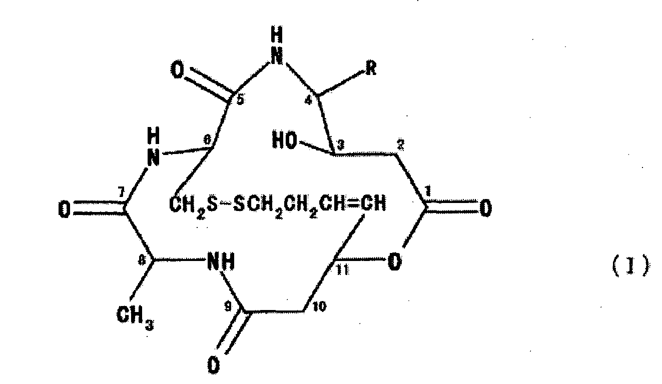 (wherein R means an isopropyl group, a sec-butyl group or an isobutyl group.)
3. The depsipeptide-containing injection solution according to claim 2, wherein the solubilizing agent is one or more members each selected from the group consisting of ethanol, methanol, 1-propanol, benzyl alcohol, chlorobutanol, propylene glycol, polyethylene glycol, N,N-dimethylacetamide, acetonitrile, polysorbate 80, povidone and castor oil.
4. The depsipeptide-containing injection solution according to claim 3, wherein the concentration of the depsipeptide compound is from 0.1 mg/mL to 100 mg/mL.
5. The depsipeptide-containing injection solution according to claim 3, wherein the concentration of the depsipeptide compound is from 0.5 mg/mL to 50 mg/mL.
6. A depsipeptide-containing injection preparation which comprises:
   a container;
   a depsipeptide-containing injection solution having a pH which is not higher than 7.0 and which is not lower than 1.0 and which is filled in said container, comprising
      (1) a depsipeptide compound having a disulfide bond in its molecule,
      (2) a solubilizing agent,
      (3) ferric chloride and/or copper sulfate and
      (4) dithiothreitol in place of (3);
   and nitrogen which replaced the space.
7. A method for preparing a depsipeptide-containing injection preparation, which comprises
   preparing a depsipeptide-containing injection preparation having a pH which is not higher than 7.0 and which is not lower than 1.0, comprising
   (1) a depsipeptide compound having a disulfide bond in its molecule,
   (2) a solubilizing agent,
   (3) ferric chloride and/or copper sulfate and
   (4) dithiothreitol in place of (3);
      then filling the preparation into a container; and
      further replacing the space with nitrogen.

The injection solution of the present invention will be further explained in detail.
The depsipeptide compound according to the present invention means a peptide where a moiety having no amino group in a constituting unit is present and, besides a peptide bond, an ester bond is present. In other words, it is a peptide constituted from an amino acid and an oxy acid. The oxy acid is a compound where an amino group in an amino acid is substituted with a hydroxy group. To be more specific, a group of compounds disclosed in JP-A-2001-354694 may be listed and, among them, Compound A mentioned in JP-A-2001-354694 is preferred.

With regard to the concentration of the depsipeptide compound having a disulfide bond in its molecule according to the present invention, it is preferred for example that the depsipeptide compound of the present invention is contained in an amount of from 0.1 mg/mL to 100 mg/mL. More preferably, it is from 0.5 mg/mL to 50 mg/mL. When the concentration is 5 mg/mL or higher, the solution may be diluted with a physiologically acceptable solution such as a physiological saline solution and subjected to a slow intravenous administration or intravenous drip administration and, in addition, it is also possible that the solution is subjected to topical administration such as intravenous injection without dilution.

In order to solubilize the depsipeptide compound which is sparingly soluble in water and, particularly, Compound A, a pharmaceutically acceptable solubilizer may be appropriately selected. To be more specific, alcohols such as ethanol, methanol, 1-propanol, benzyl alcohol, chlorobutanol, propylene glycol as well as polyethylene glycol, N,N-dimethylacetamide, acetonitrile, polysorbate 80, povidone, castor oil, etc. may be used.
Preferred ones are propylene glycol and polyethylene glycol. More preferred one is propylene glycol.

Solubility of Compound A in the solubilizer as such measured by the present inventors is 28 mg/mL (methanol), 21 mg/mL (1-propanol), 29 mg/mL (benzyl alcohol), 30 mg/mL (chlorobutanol when it is used as an auxiliary solvent for ethanol), 22 mg/mL (propylene glycol), 17 mg/mL (polyethylene glycol; M. W. = 400), 90 mg/mL (N,N-dimethylacetamide) and 29 mg/mL (acetonitrile). (All of the solvents used hereinabove are those manufactured by Kanto Kagaku.)

As to an oxidizing agent and a reducing agent used in the present invention which suppress the production of aggregates and polymers, ferric chloride, copper sulfate and dithiothreitol (DTT) are listed. A preferred one is ferric chloride which has a history of being used as an additive to commercially available injection solutions and no serious harmful action has been reported therefor. Ferric chloride and copper sulfate may be used by mixing them or each of them may be used solely. As to those oxidizing and reducing agents, commercially available ones may be used and examples thereof are iron (III) chloride (manufactured by Wako Pure Chemical Industries), copper (II) sulfate pentahydrate (manufactured by Wako Pure Chemical Industries) and dithiothreitol (manufactured by Nacalai Tesque).

The addition amount of the above substance which suppresses the production of aggregates and polymers is usually not more than 2 µg, preferably, from 0.5 µg to 2 µg per 1 mg of the depsipeptide compound having a disulfide bond in its molecule.

In the present invention, an acidic substance is used for making the pH of the injection solution not higher than 7.0 and not lower than 1.0 and there is no particular limitation therefor so far as it is usually pharmaceutically acceptable and contributes to stabilization of the depsipeptide compound. Examples thereof include hydrochloric acid, sulfuric acid, sodium hydrogen sulfite, sodium sulfite, sodium benzoate, succinic acid, ammonium acetate, lactic acid, L-aspartic acid, L-glutamic acid, benzoic acid, citric acid, tartaric acid, thioglycolic acid, glacial acetic acid, methanesulfonic acid, maleic anhydride, malic acid, phosphoric acid, ascorbic acid, gluconic acid, acetic acid and nicotinic acid. Preferred ones in view of the manufacture are hydrochloric acid, sulfuric acid and phosphoric acid. The optimum one is hydrochloric acid. Each of the acidic substances as such may be used solely or two or more thereof may be used by mixing them. The additon amount of the acidic substance per 1 mol of the depsipeptide compound of the present invention is from 0.01 mol to 0.2 mol, preferably, from 0.05 mol to 0.1 mol.

The pH of the injection solution of the present invention is preferably not higher than 7.0 and not lower than 1.0 and, more preferably, not higher than 4.0 and not lower than 1.0. The above acidic substance is compounded in such a manner that the pH of the injection solution of the present invention is finally able to be adjusted within the range upon being diluted with a physiologically acceptable solution such as a physiological saline solution.

Additives including a soothing agent such as benzyl alcohol, mepivacaine hydrochloride and xylocaine and an antiseptic agent such as benzyl alcohol, methyl p-benzoate, propyl p-benzoate, thimerosal and chlorobutanol may be added, if necessary, to the injection solution of the present invention. Additives including a hydrophilic low-molecular substance such as glucose, sodium chloride, glycine and mannitol may also be added, if necessary, for reducing the local toxicity. Further, a saccharide such as mannitol, inositol, maltose, sucrose and lactose and an amino acid such as glycine, alanine, valine and methionine may be also added thereto if necessary.
The injection solution of the present invention has an excellent compatibility with an injection solution of sodium chloride such as a physiological saline solution and with a transfusion such as saccharide transfusion and electrolyte transfusion or preferably with a saccharide transfusion and it is also possible to use the injection solution by compounding with the transfusion as such.

A method for the manufacture of the injection solution of the present invention will now be illustrated as follows.
In a process for the manufacture of the injection solution of the present invention, a substance which suppresses polymerization such as ferric chloride and water for injection solution are added to a solubilizing solvent such as propylene glycol, then a pH adjusting agent such as hydrochloric acid is added thereto for dissolution by stirring, and a depsipeptide compound such as Compound A is added thereto and dissolved with warming. After that, in order to prepare an injection preparation, an aseptic filtration is carried out, then the filtrate is filled in a predetermined container and nitrogen is introduced thereinto followed by sealing.
In order to avoid the reduction in the content of the depsipeptide compound having a disulfide bond in its molecule during the preparing stage, it is preferred that the injection solution of the present invention is manufactured by a known antiseptic operation other than the sterilization by heating. It is also possible to treat in such a manner that nitrogen gas is introduced into the prepared solution or that the space of the ampoule or the vial is filled with inert gas such as nitrogen gas so that the product does not contact with oxygen. It is further possible that, in order to prevent the light decomposition of the drug during the manufacture, the operation may be conducted in a dark place.

### BEST MODE FOR CARRYING OUT THE INVENTION

The present invention will be explained specifically by way of the following Examples but the scope of the present invention is not limited thereby.

### EXAMPLE 1

### (A 20 mg/mL injection solution containing 90% of propylene glycol, 20 µg/mL of ferric chloride and hydrochloric acid)

Compound A (22 g) was dissolved in propylene glycol with warming to make 1,000 mL. Ferric chloride (300 mg) was dissolved in a 1 mol/L aqueous solution of hydrochloric acid to make 50 mL. Compound A was completely dissolved to mix so as to make Compound A 20 mg/mL and ferric chloride 20 µg/mL and then water for injection was added thereto to make the total volume 1,000 mL whereupon an injection solution where the pH was 2.2 (5°C) was prepared.
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 98.5%.

### EXAMPLE 2

### (A 20 mg/mL injection solution containing 90% of propylene glycol, 18.5 µg/mL of copper sulfate and hydrochloric acid)

Compound A (22 g) was dissolved in propylene glycol with warming to make 1,000 mL. Copper sulfate (277.5 mg) was dissolved in a 1 mol/L aqueous solution of hydrochloric acid to make 50 mL. Compound A was completely dissolved to mix so as to make Compound A 20 mg/mL and copper sulfate 18.5 µL/mL and then water for injection was added thereto to make the total volume 1,000 mL whereupon an injection solution where the pH was 2.1 (5°C) was prepared.
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 97.1%.

### EXAMPLE 3

### (A 20 mg/mL injection solution containing 90% of propylene glycol, 11.4 µg/mL of DTT and hydrochloric acid)

Compound A (22 g) was dissolved in propylene glycol with warming to make 1,000 mL. DTT (171 mg) was dissolved in a 1 mol/L aqueous solution of hydrochloric acid to make 50 mL. Compound A was completely dissolved to mix so as to make Compound A 20 mg/mL and DTT 11.4 µL/mL and then water for injection was added thereto to make the total amount 1,000 mL whereupon an injection solution where the pH was 2.1 (5°C) was prepared.
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 95.4%.

### Comparative Example 1 (the case where none of a substance suppressing the production of polymers and an acidic substance for lowering the pH was added)

Compound A (22 g) was dissolved in propylene glycol with warming to make 1,000 mL. A propylene glycol solution where Compound A was dissolved was added to 10 mL of water for injection to make 100 mL and Compound A was completely mixed therewith to prepare an injection solution where Compound A was 20 mg/mL and the pH was 4.4 (5°C).
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 18.2%.

### Comparative Example 2 (the case where no substance which suppresses the production of polymers was added)

Compound A (22 g) was dissolved in propylene glycol with warming to make 1,000 mL. A propylene glycol solution where Compound A was dissolved was added to a solution where water for injection was added to 0.33 g of a 1 mol/L aqueous solution of hydrochloric acid to raise the volume up to 10 mL whereupon the volume was made 100 mL and Compound A was completely mixed therewith to prepare an injection solution where Compound A was 20 mg/mL and pH was 1.5 (5°C).
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 91.8%.

### Comparative Example 3 (the case where nitrogen replacement was not conducted in Comparative Example 2)

Compound A (22 g) was dissolved in propylene glycol with warming to make 1,000 mL. A propylene glycol solution where Compound A was dissolved was added to a solution where water for injection was added to 0.33 g of a 1 mol/L aqueous solution of hydrochloric acid to raise the volume up to 10 mL whereupon the volume was made 100 mL and Compound A was completely mixed therewith to prepare an injection solution where Compound A was 20 mg/mL and pH was 2.1 (5°C).
This solution was subjected to a conventional aseptic filtration and filled in a vial and the vial was stoppered to prepare an injection preparation.
Residual rate of Compound A after this preparation was stored for three days under the condition of 80°C was 48.4%.

### Comparative Example 4 (the case where o-iodobenzoic acid was added as a substance suppressing the production of polymers)

### (A 20 mg/mL injection solution containing 90% of propylene glycol, 18.4 µg/mL o-iodobenzoic acid and hydrochloric acid)

22 g was dissolved in propylene glycol with warming to make 1,000 mL. o-Iodobenzoic acid (276 mg) and 50 mL of propylene glycol were dissolved in a 1 mol/L aqueous solution of hydrochloric acid to make 100 mL. Compound A was completely dissolved, mixing was conducted so as to make Compound A 20 mg/mL and o-iodobenzoic acid 18.4 µL/mL and then water for injection was added to make the total volume 1,000 ml whereupon an injection solution in which the pH was 2.1 (5°C) was prepared.
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 91.0%.

### Comparative Example 5 (the case where sodium sulfite was added as a substance suppressing the production of polymers)

### (A 20mg/mL injection solution containing 90% of propylene glycol, 9.3 µg/mL of sodium sulfite and hydrochloric acid)

Compound A (22 g) was dissolved in propylene glycol with warming to make 1,000 mL. Sodium sulfite (139.5 mg) was dissolved in a 1 mol/L aqueous solution of hydrochloric acid to make 50 mL. Compound A was completely dissolved, mixing was conducted so as to make Compound A 20 mg/mL and sodium sulfite 9.3 µL/mL and then water for injection was added to make the total volume 1,000 ml whereupon an injection solution in which the pH was 2.1 (5°C) was prepared.
This solution was subjected to a conventional aseptic filtration and filled in a vial, the space thereof was replaced by nitrogen and the vial was stoppered to give an injection preparation.
Residual rate of Compound A after this preparation was stored for three days under the condition of 80°C was 92.8%.

### INDUSTRIAL APPLICABILITY

With regard to a depsipeptide compound having a disulfide bond in its molecule, preparations thereof up to now have been those where it is dissolved in various kinds of organic solvent followed by freeze-drying. In accordance with the present invention, however, the depsipeptide compound having a disulfide in its molecule is compounded with a substance such as ferric oxide so as to make the pH not higher than 7 whereby it is now possible to provide an injection preparation in which the manufacture and the handling of the above depsipeptide compound are easy and, further, stability is excellent for a long period of time.

## Claims

1. A depsipeptide-containing injection solution having a pH which is not higher than 7.0 and is not lower than 1.0, comprising
(1) a depsipeptide compound having a disulfide bond in its molecule,
(2) a solubilizing agent,
(3) ferric chloride and/or copper sulfate, and
(4) dithiothreitol in place of (3).

2. The depsipeptide-containing injection solution according to claim 1, wherein the depsipeptide compound is a compound represented by the formula (I): 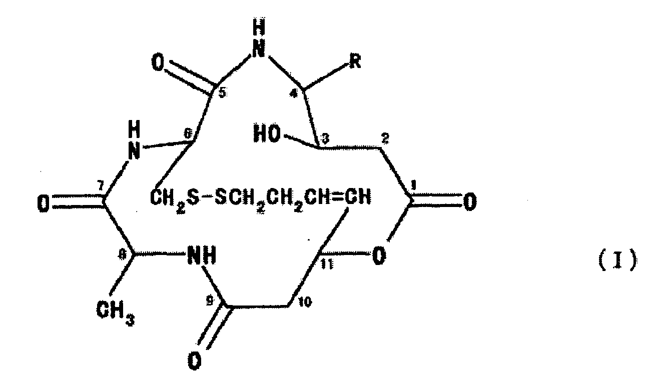 (wherein R means an isopropyl group, a sec-butyl group or an isobutyl group.)

3. The depsipeptide-containing injection solution according to claim 2, wherein the solubilizing agent is one or more members each selected from the group consisting of ethanol, methanol, 1-propanol, benzyl alcohol, chlorobutanol, propylene glycol, polyethylene glycol, N,N-dimethylacetamide, acetonitrile, polysorbate 80, povidone and castor oil.

4. The depsipeptide-containing injection solution according to claim 3, wherein the concentration of the depsipeptide compound is from 0.1 mg/mL to 100 mg/mL.

5. The depsipeptide-containing injection solution according to claim 3, wherein the concentration of the depsipeptide compound is from 0.5 mg/mL to 50 mg/mL.

6. A depsipeptide-containing injection preparation which comprises:
a container;
a depsipeptide-containing injection solution having a pH which is not higher than 7.0 and which is not lower than 1.0 and which is filled in said container, comprising
(1) a depsipeptide compound having a disulfide bond in its molecule,
(2) a solubilizing agent,
(3) ferric chloride and/or copper sulfate and
(4) dithiothreitol in place of (3);
and nitrogen which replaced the space.

7. A method for preparing a depsipeptide-containing injection preparation, which comprises
preparing a depsipeptide-containing injection preparation having a pH which is not higher than 7.0 and which is not lower than 1.0, comprising
(1) a depsipeptide compound having a disulfide bond in its molecule,
(2) a solubilizing agent,
(3) ferric chloride and/or copper sulfate and
(4) dithiothreitol in place of (3);
then filling the preparation into a container; and
further replacing the space with nitrogen.
